# EUROPEAN PATENT SPECIFICATION

(11) **EP 3 028 712 B1**
(45) Date of publication and mention of the grant of the patent: **31.05.2017**
(21) Application number: 14843115.8
(22) Date of filing: 13.06.2014
(51) Int. Cl.: A61K 38/16, A61P 25/00

(54) **USE OF RECOMBINANT LINGZHI MUSHROOM IMMUNOMODULATORY PROTEIN IN PREPARATION OF DRUGS FOR TREATING FOCAL CEREBRAL ISCHEMIA**
VERWENDUNG EINES REKOMBINANTEN IMMUNMODULATORISCHEN PROTEINS AUS LINGZHI-PILZ BEI DER HERSTELLUNG VON ARZNEIMITTELN ZUR BEHANDLUNG VON FOKALER ZEREBRALER ISCHÄMIE
UTILISATION D'UNE PROTÉINE IMMUNOMODULATRICE RECOMBINÉE DE GANODERME LUISANT DANS LA PRÉPARATION DE MÉDICAMENTS DESTINÉS AU TRAITEMENT D'UNE ISCHÉMIE CÉRÉBRALE FOCALE

(30) Priority: 06.09.2013 CN 201310402259
(43) Date of publication of application: 08.06.2016
(73) Proprietor: Zhang, Xitian, Huangpu District Shanghai 200032 (CN); Sun, Fei, Huangpu District Shanghai 200032 (CN)
(72) Inventor: LIANG, Chongyang, Shanghai 200032 (CN); ZHANG, Xitian, Shanghai 200032 (CN); SUN, Fei, Shanghai 200032 (CN)
(74) Representative: Sun, Yiming
(86) International application number: PCT/CN2014/079816
(87) International publication number: WO 2015/032233

(56) References cited:
- CN-A- 101 376 020
- CN-A- 103 463 618
- CN-A- 103 536 901
- US-A1- 2009 285 789
- ZI-YI ZHOU ET AL: "Neuroprotective effects of water-soluble Ganoderma lucidum polysaccharides on cerebral ischemic injury in rats", JOURNAL OF ETHNOPHARMACOLOGY, vol. 131, no. 1, 1 August 2010 (2010-08-01), pages 154-164, XP055262382, IE ISSN: 0378-8741, DOI: 10.1016/j.jep.2010.06.023
- DURUKAN ET AL: "Acute ischemic stroke: Overview of major experimental rodent models, pathophysiology, and therapy of focal cerebral ischemia", PHARMACOLOGY BIOCHEMISTRY AND BEHAVIOR, ELSEVIER, US, vol. 87, no. 1, 4 June 2007 (2007-06-04), pages 179-197, XP022104505, ISSN: 0091-3057
- ZHANG, YUYANG ET AL.: 'Cerebral Ischemic Damage and Inflammatory Reaction' CHINESE PHARMACOLOGICAL BULLETIN. vol. 22, no. 1, 31 January 2006, pages 5 - 9, XP055258636
- XU, LIN ET AL.: 'Protective Effect of 5-lipoxygenase Inhibitor Zileuton on Focal Cerebral Ischemia-reperfusion Injury in Rats.' CHINESE PHARMACOLOGICAL BULLETIN. vol. 22, no. 7, 31 July 2006, pages 853 - 855, XP008179472
- WANG, JUN ET AL.: 'Effects of Electroacupuncture on the Metabolism of Arachidonic Acid and Free Radical in Rats with Focal Cerebral Ischemia.' TCM RES vol. 20, no. 2, 28 February 2007, pages 14 - 17, XP008179471

## Description

### BACKGROUND OF THE PRESENT INVENTION

### Field of Invention

The present invention belongs to a field of biological pharmacy, and relates to a recombinant immunoregulatory protein LZ-8 of *ganoderma lucidum* (rLZ-8) expressed by *Pichia pastoris* for use in treating focal cerebral ischemia.

### Description of Related Arts

The *ganoderma lucidum* is a precious medicinal fungus of the traditional Chinese pharmacy, belonging to ganodermataceae of polyporales of basidiomycetes. The immunoregulatory protein of *ganoderma lucidum* is a fungal immunoregulatory protein which is separated and purified from a *ganoderma lucidum* fruit body extract firstly by Kino et al. The immunoregulatory protein of *ganoderma lucidum* comprises 110 amino acids, with N-terminal serine acetylated, has a molecular weight of 12.4 kD, and is named as LZ-8. Researches show that the LZ-8 is able to facilitate the mitosis and the immunoregulation of the cells. The rLZ-8 is extracted from PichiaPink™ strains of eukaryotic expression vectors of the rLZ-8 through screening out the stable highly-efficient secretory-expressed engineering strains, and the rLZ-8 is verified to have the same immunologic activity and biological activity as the LZ-8.

Cerebral ischemia is a kind of the cerebrovascular accidents. The cerebrovascular accident, also called cerebral apoplexy and stroke, is the common disease and the frequently-occurring disease of the middle aged and elderly people, which seriously threatens the health and the life of people. In recent years, with the continuous development of science and technology, the research scholars have deeply researched the pathogenesis of the cerebral ischemia and the cascade reaction of the brain tissue cell injury, and found that the brain tissue injury is able to be relieved through inhibiting the apoptosis and decreasing the inflammatory response and the free radical.

The rLZ-8 provided by the present invention is not only able to improve the behavior dysfunction caused by the cerebral ischemia injury, but also relieve the brain tissue injury through decreasing the inflammatory response and inhibiting the apoptosis. The present invention has a certain positive effect on the application of the rLZ-8 in developing the drug for treating the cerebral ischemia injury.

### SUMMARY OF THE PRESENT INVENTION

The present invention relates to a rLZ-8 for use in treating focal cerebral ischemia. A series of experimental measures and results show that the rLZ-8 has a significant therapeutic effect on the focal cerebral ischemia of rats. Technical solutions of the present invention are described as follows.

Taking rats as a research object, the present invention establishes rat focal cerebral ischemia models through a modified Zea Longa method, and designs six experimental groups, respectively a negative control group (sham operation group), a positive drug control group (monosialotetrahexosyl ganglioside sodium injection (GM-1)), a model group, a rLZ-8 high-dosage group (70 µg·kg⁻¹), a rLZ-8 middle-dosage group (35 µg·kg⁻¹) and a rLZ-8 low-dosage group (17.5 µg·kg⁻¹). An administration manner is designed as: an intraperitoneal injection, twice a day, wherein the same volume of physiological saline is injected into the sham operation group and the model group, for consecutive 7 days. Firstly, the therapeutic effect of the rLZ-8 on a neurological function injury is examined and graded according to a modified neurological severity scores (NSS) grading standard (16-point system). Rats of each administration group and the model group have different degrees of neurological dysfunction after modeling (the same day, about 5h after operation). The rLZ-8 high-dosage group and the rLZ-8 low-dosage group have a significant grading effect (p<0.01), which illustrates that the rLZ-8 is able to decrease a neurological function score and improve a neurological function. Moreover, the therapeutic effect of the rLZ-8 on the focal cerebral ischemia is firmly proved. Through a Hematoxylin-Eosin (HE) staining experiment, for the sham operation group, nerve cells and glial cells of a brain tissue have a normal number, and a normal morphology and distribution, with complete cell membrane, uniform cytoplasm, centrally located cell nucleus, and relatively uniformly distributed chromatin. For the model group, brain tissues are sparse; a large number of necrotic nerve cells, disappeared cell structure, pyknosis and deep staining of cell nucleus, necrotic and disintegrated cells, increased intercellular space, and a relatively larger necrotic area are observed. For the rLZ-8 high-dosage group, the brain tissues of the rats have different degrees of improvement compared with the brain tissues of the rats of the model group, showing a slight pathological change of pyknosis and dissolution of the cell nucleus and a relatively small necrotic area. An expression of ED-1 positive cells of the laboratory rats are tested by a PV-6000 two-step method immunohistochemical detection reagent. Results thereof show that, compared with the model group, an ED-1 positive cell number of the rLZ-8 high-dosage group is significantly decreased, which is able to decrease an inflammatory response caused by a cerebral ischemia injury. Apoptosis is tested by a TUNEL kit according to steps under instructions on the kit. Experimental results thereof show that an apoptotic cell number of the rLZ-8 high-dosage group is significantly lower than an apoptotic cell number of the model group, and, with an increase of a rLZ-8 concentration, the apoptotic cell number is gradually decreased, which further illustrates the therapeutic effect of the rLZ-8 on the focal cerebral ischemia.

The above series of the experimental results of the rLZ-8 for use in treating the focal cerebral ischemia show that the rLZ-8 is able to treat and relieve the neurological injury and a living state of the rats, and inhibit the apoptosis of the brain tissue to treat the cerebral ischemia injury.

These and other objectives, features, and advantages of the present invention will become apparent from the following detailed description, the accompanying drawings, and the appended claims.

### BRIEF DESCRIPTION OF THE DRAWINGS

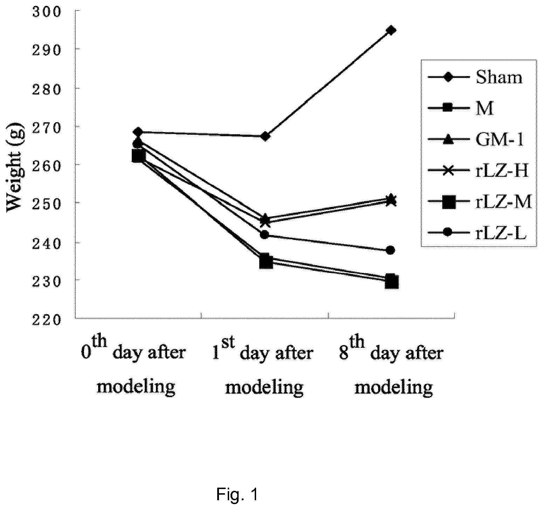

Fig. 1 is a diagram of a weight change of rats of each group according to a second example of the present invention, wherein: S is a sham operation group; M is a model group; GM-1 is a positive drug control group; H is a rLZ-8 high-dosage group; Z is a rLZ-8 middle-dosage group; and L is a rLZ-8 low-dosage group.

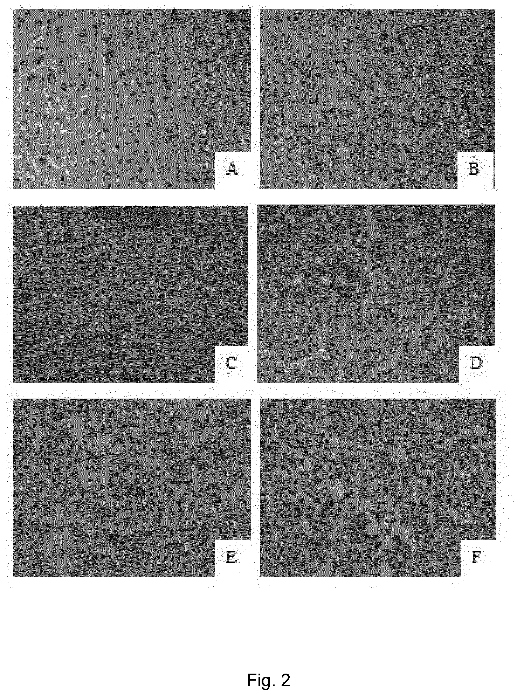

Fig. 2 shows a morphology of brain tissues of the rats of each group (HE staining, 40×) according to a third example of the present invention, wherein: A is the sham operation group; B is the model group; C is a GM-1 group; D is the rLZ-8 high-dosage group; E is the rLZ-8 middle-dosage group; and F is the rLZ-8 low-dosage group.

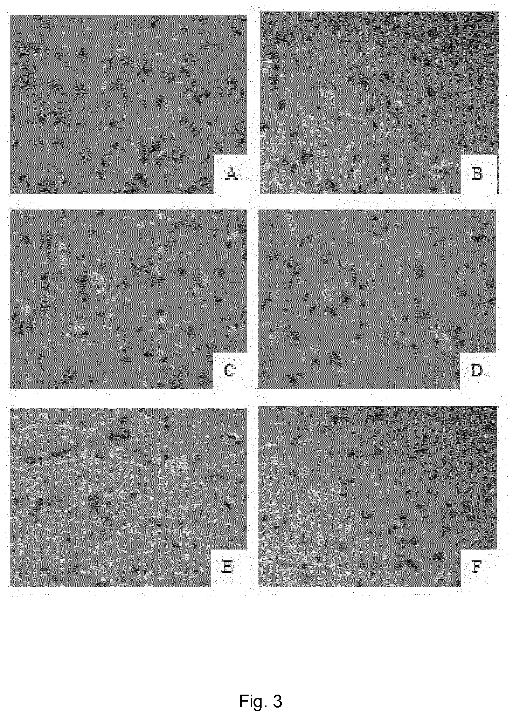

Fig. 3 shows ED-1 positive cells of each group (40×), 8 days after modeling, according to a fourth example of the present invention, wherein: A is the sham operation group; B is the model group; C is the GM-1 group; D is the rLZ-8 high-dosage group; E is the rLZ-8 middle-dosage group; and F is the rLZ-8 low-dosage group.

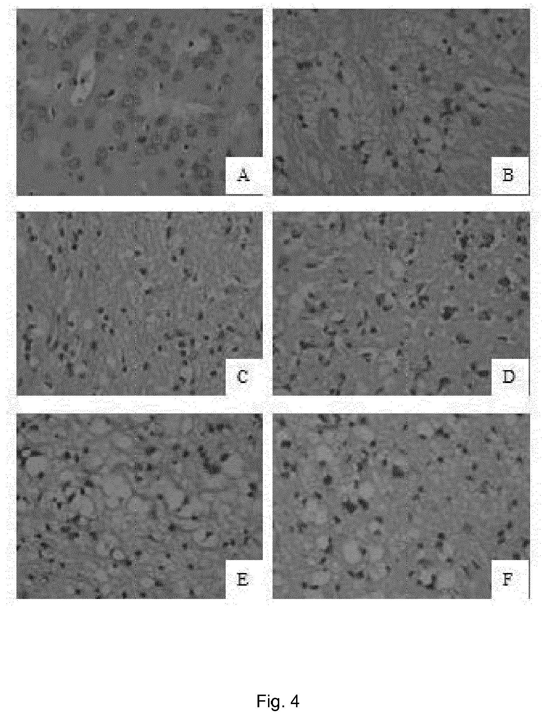

Fig. 4 shows apoptotic cells of each group (40×), 8 days after modeling, according to a fifth example of the present invention, wherein: A is the sham operation group; B is the model group; C is the GM-1 group; D is the rLZ-8 high-dosage group; E is the rLZ-8 middle-dosage group; and F is the rLZ-8 low-dosage group.

### DETAILED DESCRIPTION OF THE PREFERRED EMBODIMENT

### First example: Therapeutic effect of rLZ-8 on neurological function injury

### 1. Experimental material

Rats, having a weight of 250-300 g, half males and half females, were provided by Laboratory Animal Center of Jilin University. GM-1 was provided by Beijing Four-ring Pharmaceutical Co., Ltd. Surgical instruments.

### 2. Experimental method

Preparing rat focal cerebral ischemia models through a modified Zea Longa method; grading the rats which were fully conscious after operation according to a modified NSS grading standard (16-point system); arranging a sham operation group, a model group, a positive drug control group, a rLZ-8 high-dosage group (70 µg·kg⁻¹), a rLZ-8 middle-dosage group (35 µg·kg⁻¹) and a rLZ-8 low-dosage group (17.5 µg·kg⁻¹); and injecting intraperitoneally, twice a day, wherein the same volume of physiological saline was injected into the sham operation group and the model group, for consecutive 7 days. A positive drug in the positive drug control group was the GM-1.

### 3. Experimental results

Table 1 showed that the rats of each administration group and the model group had different degrees of neurological dysfunction after modeling (the same day, about 5h after the operation). The rats of the model group had a highest NSS, and NSS of the rLZ-8 high-dosage group and the rLZ-8 low dosage group were significantly improved (*p*<*0.01*), illustrating that the rLZ-8 is able to decrease the NSS and improve a neurological function.

**Table 1 NSS grading results of rats of each group (x̅ ± s, n=12)**

| Group | Sham | M | GM-1 | rLZ-H | rLZ-M | rLZ-L |
|---|---|---|---|---|---|---|
| 0^{th} day | 0 | 6.75±1.69 | 4.75±1.30 ^{▲▲} | 4.17±1.67^{▲▲} | 6.75±2.20 | 4.25±1.09 ^{▲▲} |
| 1^{st} day | 0 | 4.75±1.42 | 4.33±1.30 | 4.00±2.00 | 4.67±1.30 | 4.00±0.95 |
| 2^{nd} day | 0 | 3.92±0.67 | 3.92±1.38 | 3.67±1.78 | 4.00±0.85 | 3.75±0.75 |
| 3^{rd} day | 0 | 3.67±0.78 | 3.58±1.24 | 3.17±1.53 | 3.58±1.00 | 3.33±0.89 |
| 4^{th} day | 0 | 3.25±0.72 | 2.75±1.09 | 2.58±1.11 | 3.17±0.90 | 2.83±1.00 |
| 5^{th} day | 0 | 3.00±0.74 | 2.50±1.00 | 2.50±1.09 | 3.00±0.85 | 2.33±0.89 |
| 6^{th} day | 0 | 2.83±0.94 | 2.58±1.08 | 2.25±1.06 | 2.83±0.83 | 2.25±0.97 |
| 7^{th} day | 0 | 2.75±0.87 | 2.41±1.00 | 2.17±0.94 | 2.58±0.90 | 2.17±0.94 |

| | | | | | | |
|---|---|---|---|---|---|---|
| Note: comparing with the sham operation group, each administration group and the model group had significant differences, *p*<*0.01*; comparing with the model group, ^{▲}*p*<*0.05,* ^{▲▲}*p*<*0.01.* | | | | | | |

### Second example: Influence of rLZ-8 on weights of rats

### 1. Experimental method

Weighing rats before modeling; preparing rat focal cerebral ischemia models through a modified Zea Longa method; weighing the rats on the first postoperative day; and weighing the rats after consecutively administrating a drug for 7 days.

### 2. Experimental results

As shown in Table 2 and Fig. 1, before modeling, weights of the rats of each group had no significant difference; after modeling, the weights of the rats of each group were significantly decreased; and after consecutively administrating the drug for 7 days, the weights of the rats of the rLZ-8 high-dosage group were increased, illustrating that the rLZ-8 is able to reduce an injury caused by cerebral ischemia.

**Table 2 Changes of weights of rats of each group (x̅ ± s)**

| Group | 0^{th} day after modeling | 1^{st} day after modeling | 8^{th} day after modeling |
|---|---|---|---|
| Sham | 268.33±13.87 | 267.17±13.73 | 295.00±24.22 |
| M | 261.50±16.57 | 235.67±18.97^{**} | 230.17±26.49^{**} |
| GM-1 | 266.17±21.21 | 246.17±21.99 | 251.17±40.31^{*} |
| rLZ-H | 261.67±16.23 | 245.00±29.17 | 250.50±34.23^{*} |
| rLZ-M | 262.50±19.64 | 234.50±21.49^{*} | 229.67±38.85^{**} |
| rLZ-L | 265.17±31.53 | 241.83±36.97 | 237.67±38.10^{*} |

| | | | |
|---|---|---|---|
| Note: comparing with the sham operation group: ^{*}*p*<*0.05,* ^{**}*p*<*0*.*01*. | | | |

### Third example: HE staining experiment

### 1. Experimental method

Perfusing and fixing: anesthetizing a rat by chloral hydrate and fixing the rat in a supine position; processing the rat with thoracotomy to expose a heart; inserting a perfusing needle into an aorta through an apex of a left ventricle; firstly perfusing the rat quickly by physiological saline, and meanwhile, cutting off a right auricle and clamping the aorta by a hemostatic forceps; when no blood was in an effluent liquid, quickly perfusing the rat by fixative, and stopping perfusing when a body of the rat became stiff; cutting off a head to remove out a brain tissue; preparing a coronary brain section through a rat brain section mold; fixing the coronary brain section by formalin; selecting a brain section (the third brain section) which was horizontal with an optic chiasma; embedding the brain section which was horizontal with an optic chiasma by conventional paraffin and sectioning.

HE staining: (1) dewaxing the embedded brain section by xylene I and xylene II, respectively for 15 min; (2) hydrating the dewaxed brain section successively by absolute ethanol I, absolute ethanol II, 95% ethanol, 90% ethanol and 85% ethanol, respectively for 3 min, and then by distilled water for 1 min; (3) staining the hydrated brain section by hematoxylin for 5-10 min; (4) washing the stained brain section by running water to wash the hematoxylin off, for 1 min; (5) differentiating the washed brain section by 1% hydrochloric acid-ethanol for 1-3 s; (6) turning blue by the differentiated brain section, and washing by the running water for 1-3 min; (7) staining the washed brain section by eosin for 3-5 min; (8) washing the stained brain section by the distilled water for 1-3 min; (9) dehydrating and transparentizing the washed brain section, and then, sealing by neutral balsam; and (10) preliminarily observing the pathological brain section under an optical microscope.

### 2. Experimental results

As shown in Fig. 2, for the sham operation group, nerve cells and glial cells of the brain tissue had a normal number, and a normal morphology and distribution, with complete cell membrane, uniform cytoplasm, centrally located cell nucleus, and relatively uniformly distributed chromatin. For the model group, the brain tissue was sparse; a large number of necrotic nerve cells disappeared cell structure, pyknosis and deep staining of cell nucleus, necrotic and disintegrated cells; an increased intercellular space, and a relatively large necrotic area were observed. For the rLZ-8 high-dosage group, the brain tissues of the rats had different degrees of improvement compared with the brain tissues of the rats of the model group, showing a slight pathological change of pyknosis and dissolution of the cell nucleus, and a relatively small necrotic area.

### Fourth example: Decreasing inflammatory response by rLZ-8

### 1. Experimental reagent

The rLZ-8 high-dosage group, the rLZ-8 middle-dosage group and the rLZ-8 low-dosage group were provided and prepared by physiological saline to respectively have a concentration of 70 µg·ml⁻¹, 35 µg·ml⁻¹ and 17.5 µg·ml⁻¹. ED-1 antibody was provided by Wuhan Boster Biological Technology Co., Ltd., and PV-6000 two-step method immunohistochemical detection reagent was provided by Beijing Zhongshan Golden Bridge Biotechnology Co., Ltd.

### 2. Experimental group

The sham operation group, the model group, the positive drug control group, the rLZ-8 high-dosage group (70 µg·kg⁻¹), the rLZ-8 middle-dosage group (35 µg·kg⁻¹) and the rLZ-8 low-dosage group (17.5 µg·kg⁻¹) were provided.

### 3. Experimental method

After being embedded by conventional paraffin and sectioned, a brain tissue paraffin section was tested through an immunologic tissue chemical staining method to test an ED-1 positive cell expression thereof. (1) baking the brain tissue paraffin section: arranging the brain tissue paraffin section in an oven at 60 °C for 30-60 min and cooling naturally; (2) dewaxing the baked brain tissue paraffin section: arranging the baked brain tissue paraffin section successively in xylene I and exlene II, respectively for 15 min; (3) hydrating: arranging the dewaxed brain tissue section successively in absolute ethanol I, absolute ethanol II, 90% ethanol, 70% ethanol and distilled water, respectively for 2 min; (4) preprocessing: according to special requirements of the applied ED-1 antibody, preprocessing the hydrated brain tissue section, wherein antigen is processed by 0.01 M citrate buffer solution (pH 6.0) for thermal remediation; (5) incubating the preprocessed brain tissue section in 3% H₂O₂ deionized water for 10 min, so as to block endogenous peroxidase, and washing by phosphate buffered saline (PBS) for 3 times, 5 min every time; (6) adding the ED-1 antibody to the washed brain tissue section, staying for a night at 4 °C, and washing by the PBS for 3 times, 5 min every time; (7) adding general immunoglobulin G (IgG) antibody-horseradish peroxidase (HRP) polymer to the washed brain tissue section, incubating at a room temperature for 15 min, and washing by the PBS for 3 times, 5 min every time; (8) colorating the washed brain tissues section by DAB solution (9) washing the colorated brain tissue section by the distilled water, staining by hematoxylin, dehydrating, transparentizing, and sealing by neutral balsam; and (10) observing the sealed brain tissue section under an optical microscope.

### 4. Experimental results

As showed in Table 3 and Fig. 3, compared with the model group, an ED-1 positive cell number of the rLZ-8 high-dosage group significantly decreased, which decreased an inflammatory response caused by a cerebral ischemia injury.

**Table 3 ED-1 positive cell number of each group after modeling for 8 days (x̅±s)**

| Group | ED-1 |
|---|---|
| Sham | 8.73±2.93 |
| M | 24.53±4.04^{**} |
| GM-1 | 13.87±2.39*^{▲} |
| rLZ-H | 15.27±1.42*^{▲} |
| rLZ-M | 20.00±2.62^{#**} |
| rLZ-L | 19.00±2.31^{**} |

| | |
|---|---|
| Note: comparing with the sham operation group, **p*<*0.05*, ^{**}*p*<*0.01*; comparing with the model group, ^{▲}*p*<*0.05*; comparing with the GM-1 group, ^{#}*p*<*0.05.* | |

### Fifth example: Test of apoptosis by TUNEL kit

### 1. Experimental reagent

The rLZ-8 high-dosage group, the rLZ-8 middle-dosage group and the rLZ-8 low-dosage group were provided and prepared by physiological saline to respectively have a concentration of 70 µg·ml⁻¹, 35 µg·ml⁻¹ and 17.5 µg·ml⁻¹. TUNEL kit.

### 2. Experimental group

The sham operation group, the model group, the positive drug control group, the rLZ-8 high-dosage group (70 µg·ml⁻¹), the rLZ-8 middle-dosage group (35 µg·ml⁻¹) and the rLZ-8 low-dosage group (17.5 µg·ml⁻¹) were provided.

### 3. Experimental method

After being embedded by conventional paraffin and sectioned, a brain tissue paraffin section was processed according to instructions of the TUNEL kit. (1) processing the brain tissue paraffin section with conventional dewaxing and hydration; (2) processing the brain tissue section with trypsin, at 37 °C, in a dark wet kit for 15-60 min; (3) washing the processed brain tissue section by PBS for twice, 5 min every time; (4) adding 50 µl TUNEL reaction mixture (for a negative control group, adding 50 µl dUTP solution marked by fluorescein) on a specimen of the washed brain tissue section, and reacting in the dark wet kit at 37 °C for 1 h; (5) washing by the PBS for 3 times, 5 min every time; (6) adding 50 µl Converter-POD on the specimen, and reacting in the dark wet kit at 37 °C for 30 min; (7) washing the reacted brain tissue section by the PBS for 3 times, 5 min every time; (8) adding 50-100 µl DAB solution on the washed brain tissue section for coloration; (9) washing the colorated brain tissue section by distilled water, staining by hematoxylin, dehydrating, transparentizing, and sealing by neutral balsam; and (10) observing the sealed brain tissue section under an optical microscope.

### 4. Experimental results

As shown in Table 4 and Fig. 4, an apoptotic cell number of each rLZ-8 group was significantly lower than an apoptotic cell number of the model group; and, with an increase of a rLZ-8 concentration, the apoptotic cell number gradually decreased.

**Table 4 Apoptotic cell number of each group after modeling for 8 days (x̅ ± s)**

| Group | TUNEL |
|---|---|
| Sham | 7.87±0.12 |
| M | 45.20±3.81^{**} |
| GM-1 | 23.10±2.47^{**▲▲} |
| rLZ-H | 18.80±4.08^{**▲▲} |
| rLZ-M | 33.20±2.16^{**▲▲##} |
| rLZ-L | 39.27±4.80^{**##} |

| | |
|---|---|
| Note: comparing with the sham operation group, ^{**}*p*<*0.01*; comparing with the model group, ^{▲▲}*p*<*0.01*; comparing with the GM-1 group, ^{##}*p*<*0.01.* | |

One skilled in the art will understand that the embodiment of the present invention as shown in the drawings and described above is exemplary only and not intended to be limiting.

It will thus be seen that the objects of the present invention have been fully and effectively accomplished. Its embodiments have been shown and described for the purposes of illustrating the functional and structural principles of the present invention and is subject to change without departure from such principles.

## Claims

1. A recombinant immunoregulatory protein IZ-8 of *ganoderma lucidum* (rLZ-8) for use in treating focal cerebral ischemia.

2. The recombinant immunoregulatory protein LZ-8 for use according to claim 1, wherein the recombinant immunoregulatory protein LZ-8 of the *ganoderma lucidum* decreases a neurological severity score caused by a cerebral ischemia injury.

3. The recombinant immunoregulatory protein LZ-8 for use according to claim 1, wherein the recombinant immunoregulatory protein LZ-8 of the *ganoderma lucidum* decreases an ED-1 positive cell number, decreases an inflammatory response and treats a cerebral ischemia injury.

4. The recombinant immunoregulatory protein IZ-8 for use according to claim 1, wherein the recombinant immunoregulatory protein LZ-8 of the *ganoderma lucidum* decreases an apoptotic cell number and inhibits apoptosis of a brain tissue.

5. The recombinant immunoregulatory protein LZ-8 for use according to claim 1, wherein core ingredients of the drug comprise the recombinant immunoregulatory protein of the *ganoderma lucidum* (rLZ-8), as recited in claim 1, and a pharmaceutically acceptable adjuvant.

6. The recombinant immunoregulatory protein IZ-8 for use according to claim 1, wherein administration methods of the drug comprise oral administration and parenteral administration; the oral administration comprises oral liquid, tablet, pill and capsule; the parenteral administration comprises externally applied agent and injection.

## Patentansprüche

1. Rekombinantes immunregulatorisches Protein IZ-8 von *Ganoderma lucidum* (rLZ-8) zur Verwendung in der Behandlung von fokaler zerebraler Ischämie.

2. Rekombinantes immunregulatorisches Protein LZ-8 zur Verwendung nach Anspruch 1, wobei das rekombinante immunregulatorische Protein LZ-8 von *Ganoderma lucidum* einen neurologischen Severity-Score vermindert, der durch eine zerebrale ischämische Schädigung verursacht ist.

3. Rekombinantes immunregulatorisches Protein LZ-8 zur Verwendung nach Anspruch 1, wobei das rekombinante immunregulatorische Protein LZ-8 von *Ganoderma lucidum* eine ED-1-positive Zellzahl vermindert, eine Entzündungsreaktion vermindert und eine zerebrale ischämische Schädigung behandelt.

4. Rekombinantes immunregulatorisches Protein IZ-8 zur Verwendung nach Anspruch 1, wobei das rekombinante immunregulatorische Protein LZ-8 von *Ganoderma lucidum* eine apoptotische Zellzahl vermindert und Apoptose eines Hirngewebes inhibiert.

5. Rekombinantes immunregulatorisches Protein LZ-8 zur Verwendung nach Anspruch 1, wobei die Kernbestandteile des Arzneimittels das rekombinante immunregulatorische Protein von *Ganoderma lucidum* (rLZ-8) gemäß Anspruch 1 und ein pharmazeutisch annehmbares Adjuvans umfassen.

6. Rekombinantes immunregulatorisches Protein IZ-8 zur Verwendung nach Anspruch 1, wobei das Verabreichungsverfahren des Arzneimittels eine orale Verabreichung und eine parenterale Verabreichung umfasst; wobei die orale Verabreichung eine orale Flüssigkeit, eine Tablette, Pille und Kapsel umfasst; wobei die parenterale Verabreichung äußerlich aufgetragene Mittel und Injektion umfasst.

## Revendications

1. Protéine immunorégulatrice recombinante LZ-8 de *Ganoderma lucidum* (rLZ-8) pour son utilisation dans le traitement de l'ischémie cérébrale focale.

2. Protéine immunorégulatrice recombinante LZ-8 pour son utilisation selon la revendication 1, dans laquelle la protéine immunorégulatrice recombinante LZ-8 de *Ganoderma lucidum* réduit un score de gravité neurologique provoqué par une lésion ischémique cérébrale.

3. Protéine immunorégulatrice recombinante LZ-8 pour son utilisation selon la revendication 1, dans laquelle la protéine immunorégulatrice recombinante LZ-8 de *Ganoderma lucidum* réduit un nombre de cellules ED-1 positives, réduit une réponse inflammatoire et traite une lésion ischémique cérébrale.

4. Protéine immunorégulatrice recombinante LZ-8 pour son utilisation selon la revendication 1, dans laquelle la protéine immunorégulatrice recombinante LZ-8 de *Ganoderma lucidum* réduit un nombre de cellules apoptotiques et inhibe l'apoptose d'un tissu cérébral.

5. Protéine immunorégulatrice recombinante LZ-8 pour son utilisation selon la revendication 1, dans laquelle les principaux ingrédients du médicament comprennent la protéine immunorégulatrice recombinante de *Ganoderma lucidum* (rLZ-8), selon la revendication 1, et un adjuvant pharmaceutiquement acceptable.

6. Protéine immunorégulatrice recombinante LZ-8 pour son utilisation selon la revendication 1, dans laquelle les procédés d'administration du médicament comprennent l'administration orale et l'administration parentérale ; l'administration orale comprend un liquide, un comprimé, une pilule et une gélule à administrer par voie orale ; l'administration parentérale comprend un agent d'application externe et une injection.
